(19) Europäisches Patentamt
European Patent Office
Office européen des brevets

(11) Publication number: 0 309 971 A2

# (12) EUROPEAN PATENT APPLICATION

(21) Application number: 88115845.5

(22) Date of filing: 27.09.88

(51) Int. Cl.[4]: C07C 129/12 , A61K 31/16

(30) Priority: 30.09.87 JP 244260/87

(43) Date of publication of application: 05.04.89 Bulletin 89/14

(84) Designated Contracting States: DE FR GB IT

(71) Applicant: ZAIDAN HOJIN BISEIBUTSU KAGAKU KENKYU KAI
14-23, Kamiosaki 3-chome
Shinagawa-ku Tokyo(JP)

(72) Inventor: Takeuchi, Tomio
1-11, Higashi-gotanda 1-chome
Shinagawa Tokyo(JP)
Inventor: Tomiyoshi, Tsugio
1-302, Shimo 3-17
Kita-ku Tokyo(JP)
Inventor: Saino, Tetsushi
14-101, Hachiohji 5-11
Yono-shi Saitama-ken(JP)
Inventor: Takahashi, Katsutoshi
8-512, Kamiya 3-10
Kita-ku Tokyo(JP)
Inventor: Nakamura, Teruya
831-6, Nomura-cho
Kusatsu-shi Shiga-ken(JP)

(74) Representative: Türk, Gille, Hrabal
Bruckner Strasse 20
D-4000 Düsseldorf 13(DE)

## (54) New spergualin-related compound and pharmaceutical composition.

(57) The present invention relates to new spergualin-related compounds having an immunopotentiating effect and represented by the general formula [I]:

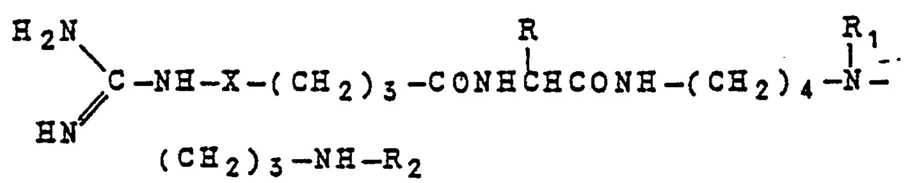

[I]

wherein X represents $-(CH_2)_{3\sim5}-$ or

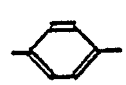

,

EP 0 309 971 A2

R represents -H or $-CH_2-OH$ and $R_1$ and $R_2$ each represents a residue formed by removing a hydroxyl group from the carboxyl group of an amino acid or peptide with the proviso that both of $R_1$ and $R_2$ cannot represent

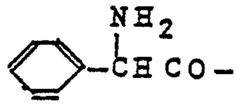

at the same time,
and pharmacologically acceptable salts thereof.

# NEW SPERGUALIN-RELATED COMPOUNDS AND PHARMACEUTICAL COMPOSITIONS

## Background of the Invention:

Spergualin is an antibiotic found by Umezawa (see British Patent No. 2084999). Heretofore various spergualin-related compounds have been prepared by the inventors (see, for example, USP 4,518,532, USP 4,556,735).

Although various immunopotentiators and immunosuppressants have been used as immunomodulators, they are yet unsatisfactory. Under these circumstances, the development of new immunomodulators is demanded.

## Summary of the Invention:

The compounds of the present invention have an immunomodulating effect so that they are expected as medicines for manmarian having immunodeficiency.

After intensive investigations, the inventors have found that new spergualin-related compounds of the following general formula [I]:

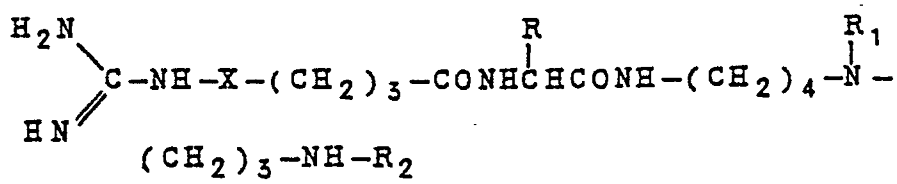

wherein x represents $-(CH_2)_{3\sim5}-$ or

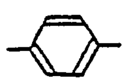 ,

R represents -H or $-CH_2-OH$ and $R_1$ and $R_2$ each represent a residue formed by removing a hydroxyl group from the carboxyl group of an amino acid or peptide with the proviso that both of $R_1$ and $R_2$ cannot represent

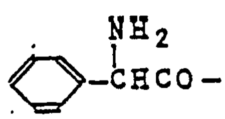

at the same time,
and pharmacologically acceptable salts thereof have an excellent immunomodulating effect such as immune enhancement. The present invention has been completed on the basis of this finding.

Therefore the present invention relates to new spergualin-related compounds and pharmaceutical compositions containing them.

$R_1$ and $R_2$ in the general formula (I) of the present invention include residues formed by removing a hydroxyl group from the carboxyl group of, for example, the following amino acids and peptides. The configuration of the residues of the amino acids excluding glycine, β-alanine and γ-aminobutyric acid is of L, D or DL configuration.

(1) Amino acids:

The amino acids include, for example, alanine, arginine, ornithine, aspartic acid, asparagine, cysteine,

cystine, glutamic acid, glutamine pyroglutamic acid, glycine, histidine, lysine, proline, hydroxyproline, isoleucine, leucine, methionine, phenylalanine, phenyl-substituted phenylalanine, serine, threonine, tryptophan, homoserine, tyrosine, valine, phenylglycine, p-hydroxyphenylglycine, 4-hydroxymethyl-3-hydroxyphenylglycine, β-alanine, γ-aminobutyric acid and 3-amino-2-hydroxy-4-phenylburyric acid.

(2) Peptides:

The peptides are preferably di- or tripeptides comprising one of the above-mentioned amino acids (1) or condensation products of a combination of 2 or 3 amino acids. They include, for example, alanylalanine, leucylleucine, valylvaline, phenylalanylphenylalanine, tyrosyltyrosine, phenylglycylphenylglycine, glycylglycine, isoleucylisoleucine, leucylphenylalanine, phenylalanylleucine, leucylphenylglycine, phenylglycylleucine, glycylglycylglycine, phenylglycylphenylglycylphenylglycine, phenylalanylphenylalanylphenylalanine and leucylleucylleucine.

Preferred amino acids and peptides include, for example, phenylglycine, phenylalanine, leucine, aspartic acid, tryptophan, alanine and peptides formed by condensation of 2 or 3 of these amino acids.

Typical examples of the groups $R_1$ and $R_2$ are as follows:

(1) groups of the formula:

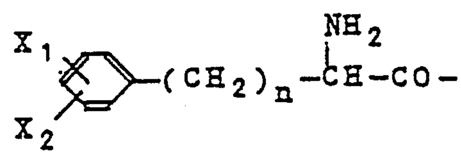

wherein n represents 0 or 1, $X_1$ represents -H or -OH and $X_2$ represents -H or $-CH_2OH$,

(2) groups of the formula:

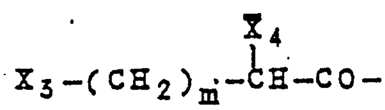

wherein m represents an integer of 0 to 4, $X_3$ represents -H, -COOH, -OH, $-NH_2$ or $-CONH_2$ and $X_4$ represents -H or $-NH_2$, with the proviso that at least one of $X_3$ and $X_4$ represents $-NH_2$,

(3) groups of the formula:

$H-(A)_y-$

wherein y represents 1 or 2, and A represents

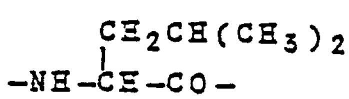

or

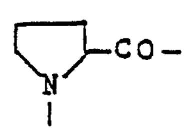

,

with the proviso that when y is 2, both groups A are bonded to each other through a peptide bond, and

(4) a group of the formula:

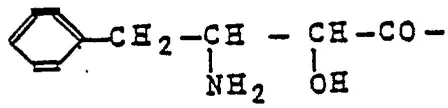

Among the compounds of the above general formula [I], preferred are those wherein X represents

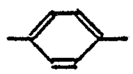 ,

R represents $-CH_2OH$, and $R_1$ and $R_2$ may be the same or different from each other and represent

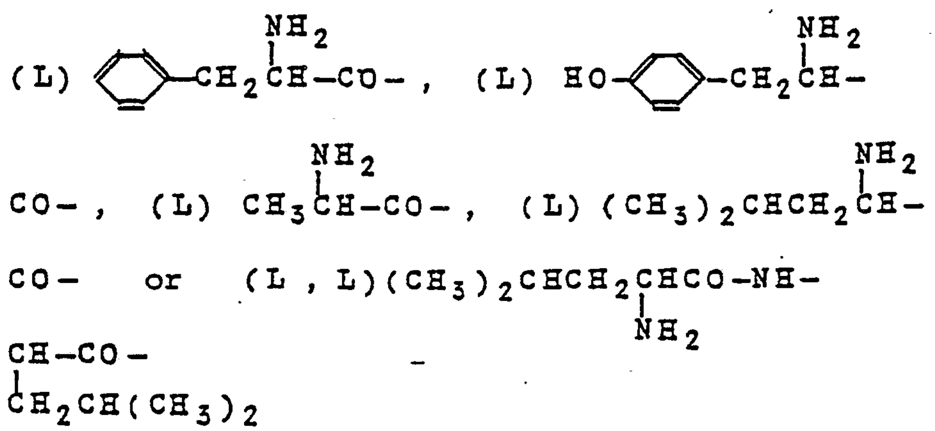

and pharmacologically acceptable salts of them.

The new spergualin-related compounds of the general formula (I) form salts with acids. The acids usable for forming the salts may be both inorganic and organic acids so far as they are non-toxic. Though the inorganic acids are not particularly limited, they are preferably hydrochloric, sulfuric, nitric and phosphoric acids. Though the organic acids are also not particularly limited, they are preferably acetic, propionic, succinic, fumaric, maleic, malic, tartaric, glutaric, citric, benzenesulfonic, toluenesulfonic, methanesulfonic, ethanesulfonic, propanesulfonic, aspartic and glutamic acids.

Examples of the compounds of the general formula [I]:

The compounds of the general formula [I] of the present invention include, for example, those listed in the following Table 1.

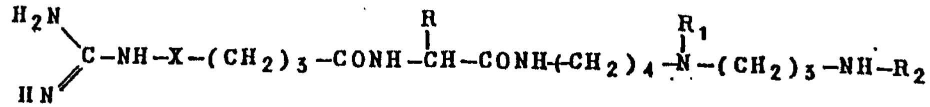

Table 1

| Compound No. | X | R | $R_1$ | $R_2$ | Configuration of $R_2$ |
|---|---|---|---|---|---|
| 1 | 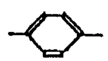 | $-CH_2-OH$ | 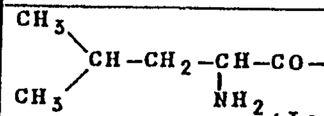 (Leu) | 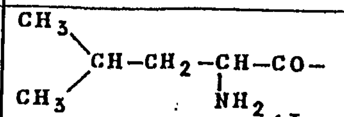 (Leu) | L |
| 2 | 〃 | 〃 | 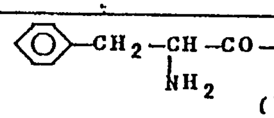 (Phe) | 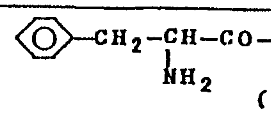 (Phe) | L |
| 3 | 〃 | 〃 | 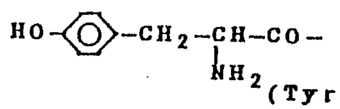 (Tyr) | 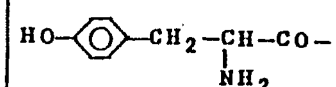 (Tyr) | L |
| 4 | 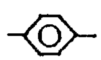 | 〃 | 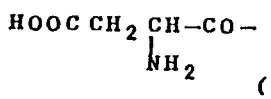 (Asp) | 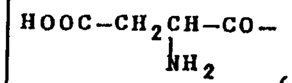 (Asp) | L |
| 5 | 〃 | 〃 | 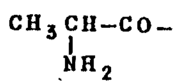 (Ala) | 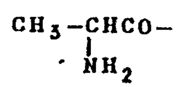 (Ala) | L |
| 6 | 〃 | 〃 | 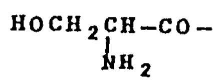 (Ser) | 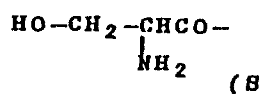 (Ser) | L |

Table 1 (cont'd)

| Compound No. | X | R | $R_1$ | $R_2$ | Configuration of $R_2$ |
|---|---|---|---|---|---|
| 7 | 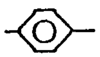 | $-CH_2-OH$ | 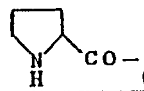 (Pro) | 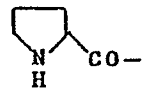 (Pro) | L |
| 8 | 〃 | 〃 | $NH_2CH_2CH_2CO-$ (β-Ala) | $NH_2CH_2CH_2CO-$ (β-Ala) | |
| 9 | 〃 | 〃 | $NH_2CH_2CO-$ (Gly) | $NH_2CH_2CO-$ (Gly) | |
| 10 | 〃 | 〃 | 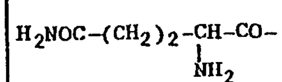 (Gln) | 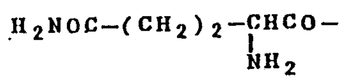 (Gln) | L |
| 11 | 〃 | 〃 | 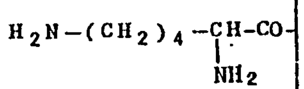 (Lys) | 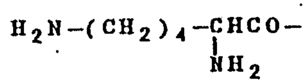 (Lys) | L |
| 12 | 〃 | 〃 | 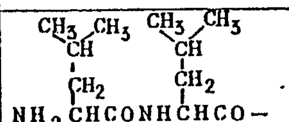 (Leu-Leu) | 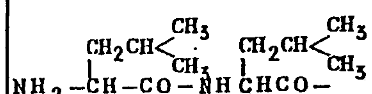 (Leu-Leu) | L, L |
| 13 | 〃 | H | 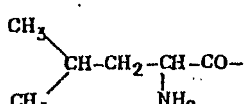 (Leu) | 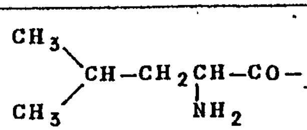 (Leu) | L |

Table 1 (cont'd)

| Compound No. | X | R | $R_1$ | $R_2$ | Configuration of $R_2$ |
|---|---|---|---|---|---|
| 14 | $-(CH_2)_3-$ | H | 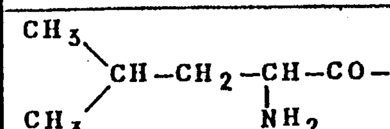 (Leu) | 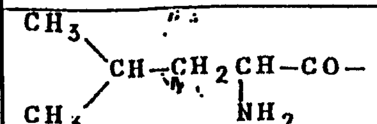 (Leu) | L |
| 15 | 〃 | $-CH_2OH$ | 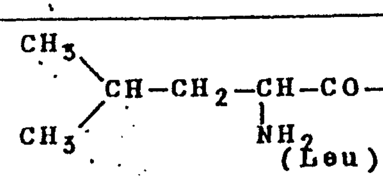 (Leu) | 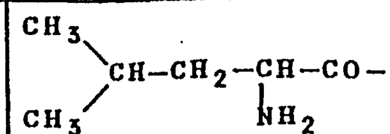 (Leu) | L |

The compounds of the general formula [I] of the present invention are prepared by removing the protective group of a protected compound of the following general formula [II]:

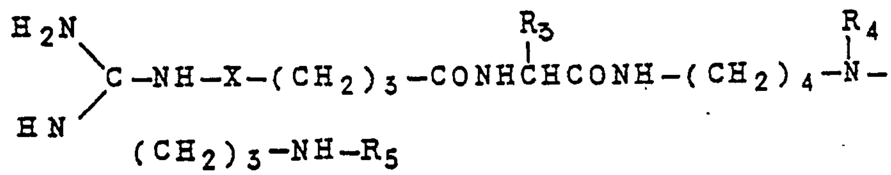

wherein X represents $-(CH_2)_{3\sim5}-$ or

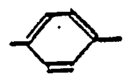 ,

$R_3$ represents -H or $-CH_2-O-Y$ (Y being -H or a protective group for the hydroxyl group), and $R_4$ and $R_5$ each represent a residue formed by removing a hydroxyl group from the carboxyl group of an amino acid or peptide having a protected amino group (a side chain of the residue being either protected or non-protected), with the proviso that both $R_4$ and $R_5$ do not represent an N-protected phenylglycyl group at the same time.

The protective group can be removed by, for example, reduction, acid decomposition or hydrolysis.

The reaction is conducted usually in an inert solvent at a temperature of -60 °C to the boiling point of the solvent, preferably about -50 °C to 100 °C. The inert solvents include water and hydrophilic organic solvents such as lower alcohols, e.g., methanol and ehtnaol, ketones, e.g., acetone and methyl ethyl ketone, amides, e.g., dimethylformamide and dimethylacetamide, cyclic ethers, e.g., tetrahydrofuran and dioxane, lower fatty acids, e.g., acetic acid and trifluoroacetic acid, liquid ammonia, and liquid hydrogen fluoride.

After removing the protective group, the new spergualin-related compound of the general formula (I) can be isolated as follows: in case the protective group has been removed by catalytic reduction with palladium black, the catalyst is separated by filtration, the filtrate is concentrated under reduced pressure and the residue is purified by a known purification process with Cm-Sephadex® ($Na^+$) or Sephadex® LH-20 [see T. Takeuchi et al., J. Antibiotics, 34, 1619 (1981)], and in case the protective group has been removed with trifluoroacetic acid, the reaction mixture is concentrated under reduced pressure and the residue is purified by the above-mentioned known purification process to isolate the intended compound.

By the above-mentioned purification process, the new spergualin-related compound of the general formula [I] is obtained in the form of its hydrochloride. This salt can be converted into a salt with another acid by, for example, dissolving the hydrochloride in water, passing the aqueous solution through a strongly basic ion exchange resin to collect fractions containing the intended compound, neutralizing it with an intended acid, an aqueous solution thereof or a solution thereof in a hydrophilic organic solvent such as methanol, ethanol, acetone, tetrahydrofuran or dioxane and evaporating the neutralized solution to dryness under reduced pressure, or distilling off the organic solvent, in case the organic solvent is used, under reduced pressure and freeze-drying the residue. Another process for converting the salt into a salt with another acid comprises adding an aqueous solution of silver hydroxide or silver oxide to the hydrochloride of the compound of the general formula (I) to neutralize it, removing precipitated silver chloride by filtration, adding an intended acid to the filtrate to form a salt and freeze-drying the product.

The intended product of the present invention prepared by the above-described process may be a hydrate depending on the treating conditions.

The preparation of the protected spergualin-related compounds of the general formula [II] which are starting compounds of the present invention will now be described.

(a) Preparation of the compounds of the general formula [II] wherein $R_4$ and $R_5$ each represent an N-protected amino acid residue or an N-protected peptide residue:

A compound of the general formula [III]:

$$NH_2-(CH_2)_4-\underset{\underset{R'_4}{|}}{N}-(CH_2)_3-NH-R''_5 \qquad [III]$$

wherein $R'_4$ and $R''_5$ each represent an N-protected amino acid residue or an N-protected peptide residue, is condensed with a protected amino acid of the general formula [IV]:

$R_3$

$P_2$-NH CH COOH [IV]

wherein $P_2$ represents a protective group and $R_3$ is as defined above, or a reactive derivative thereof to form a compound of the general formula [V]:

$R_3$

$P_2$-NH CH CONH-$(NH_2)_4$- N - $(CH_2)_3$-NH-$R''_5$ [V]

$R'_4$

wherein $P_2$, $R_3$, $R'_4$ and $R''_5$ are as defined above, the protective group $P_2$ is selectively removed and the compound thus obtained is condensed with an ω-guanidino fatty acid of the general formula [VI]:

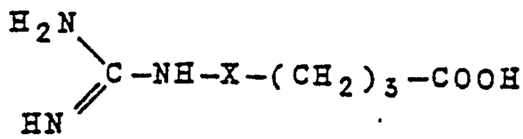

[VI]

wherein X is as defined above, or a reactive derivative thereof to give a compound of the general formula [II], wherein $R_4$ and $R_5$ each represent an N-protected amino acid residue or an N-protected peptide residue.

The compounds of the general formula [III] can be prepared by an ordinary process such as a process wherein 1 mol of an N-protected spermidine of the general formula [VII]:

$P_1$-NH-$(CH_2)_4$-NH-$(CH_2)_3NH_2$ [VII]

wherein $P_1$ represents a protective group, is condensed with 1 mol of a reactive derivative prepared from an N-protected amino acid of the general formula [VIII]:

$R''_5$ - OH [VIII]

wherein $R''_5$ is as defined above, and 1,3-thiazolidine-2-thione, the condensate thus obtained is reacted with an N-protected amino acid of the general formula [IX]:

$R'_4$- OH [IX]

wherein $R'_4$ represents a residue formed by removing -OH from an amino acid (a side chain of which may be protected) having a protected amino group, and the protective group Pl is selectively removed.

When $R'_4$ and $R''_5$ in the above formula are the same, the compound can be prepared by, in addition to the above-described process, a process wherein 1 mol of an N-protected spermidine of the general formula [VII] is reacted with at least 2 mol of a protected amino acid of the above general formula [VIII] or [IX] or a reactive derivative thereof and the protective group $P_1$ is selectively removed.

The N-protected spermidine of the general formula [VII] can be prepared also by reacting a compound of the general formula [X]:

$P_1$-NH$(CH_2)_4NH_2$ [X]

wherein $P_1$ is as defined above, with acrylonitrile and then reducing the nitrile group.

The condensation in the above preparation processes (a) can be conducted by an ordinary process employed in the condensation of peptide bonds. These processes include, for example, the carbodiimide method wherein dicyclohexylcarbodiimide or 1-ethyl-3-(3-dimethylaminopropyl)carbodiimide is used, the azide method wherein a hydrazide is used, the mixed acid anhydride method wherein ethyl chlorocarbonate or isobutyl chlorocarbonate is used, the active ester method wherein a cyanomethyl ester, vinyl ester, substituted or unsubstituted phenyl ester, thiophenyl ester or hydroxysuccinimide ester is used, the O-acylhydroxylamine derivative method wherein acetoxime or cyclohexanone oxime is used, the N-acyl

compound method wherein carbonyldiimidazole or the like is used and the carboxylic acid activation method wherein 1,3-thiazolidine-2-thione is used. The solvents usable in the condensation are those ordinarily used in the condensation of peptide bonds. They include, for example, ethers such as diethyl ether, tetrahydrofuran and dioxane, esters such as ethyl acetate, ketones such as acetone and methyl ethyl ketone, halogenated hydrocarbons such as methylene chloride and chloroform, amides such as dimethylformamide and dimethylacetamide, and nitriles such as acetonitrile. These solvents are used either singly or in the form of a solvent mixture with water when they are miscible with water.

The protective groups for the amino group usable in the present invention include, for example, a benzyloxycarbonyl group, substituted benzyloxycarbonyl groups such as a p-methoxybenzyloxycarbonyl group, a t-butyloxycarbonyl group, a t-amyloxycarbonyl group, a formyl group, a trityl group and an O-nitrophenylsulfenyl group.

Among the protective groups for the side chains of the amino acids, those for the carboxyl group include lower alkyl, t-butyl, benzyl and substituted benzyl groups, those for the hydroxyl group include t-butyl and benzyl groups, those for the mercapto group include benzyl and p-methoxybenzyl groups, those for the imidazole group include benzyloxycarbonyl, benzyl and tosyl groups and those for the guanidine group include nitro, tosyl and t-butyloxycarbonyl groups. However, the protective groups are not limited to those mentioned above.

Typical examples of the compounds of the general formula [II] usable as the starting material are shown in Table 2. Symbols for the amino acid residues, protective groups and the amino acid residues having a protective group are as shown below. The configuration of the amino acid excluding glycine, β-alanine and γ-aminobutyric acid is of L, D or DL configuration.

Amino acid residues:

Ala: alanyl residue,
Leu: leucyl residue,
Phe: phenylalanyl residue,
Asp: aspartyl residue,
Asn: asparaginyl residue,
Lys: lysyl residue,
PhG: phenylglycyl residue,
Pro: prolyl residue,
Tyr: tyrosyl residue,
Ser: seryl residue,
β-Ala: β-alanyl residue,
AHPA: 3-amino-2-hydroxy-4-phenylbutyryl residue,
Gly: glycyl residue,
Glu: glutamyl residue, and
γ-ABA: γ-aminobutyryl residue.

Protecting groups:

Z: benzyloxycarbonyl group,
Boc: t-butyloxycarbonyl group,
pMZ: p-methoxybenzyloxycarbonyl group, and
Aoc: t-amyloxycarbonyl group.

Amino acid residues having a protective group:

Asp(OBzl): β-benzylaspartyl,
Asp(OBu$^t$): β-t-butylaspartyl,
Ser(Bzl): O-benzylseryl,
Ser(Bu$^t$): O-t-butylseryl,
Z
|
Lys: ε-benzyloxycarbonyllysyl,

Boc
Lys: ε-t-butyloxycarbonyllysyl,
pMz
Lys: ε-p-methoxybenzyloxycarbonyllysyl, and
Tyr($Bu^t$): O-t-butyltyrosyl.

Table 2. Typical compounds of the general formula [II]:

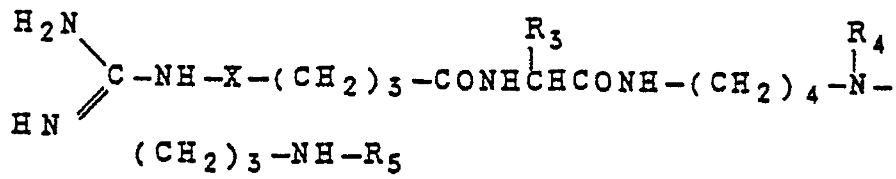

| X | $R_3$ | $R_4$ | $R_5$ |
|---|---|---|---|
| 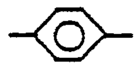 | $-CH_2OH$ | Z-Leu- | Z-Leu- |
| ″ | ″ | Boc-Leu- | Boc-Leu- |
| ″ | ″ | pMz-Leu- | pMz-Leu- |
| ″ | ″ | Z-Phe- | Z-Phe- |
| ″ | ″ | Boc-Phe- | Boc-Phe- |
| ″ | ″ | pMz-Phe- | pMz-Phe- |
| ″ | ″ | Z-Tyr- | Z-Tyr- |
| ″ | ″ | Aoc-Tyr($Bu^t$)- | Aoc-Tyr($Bu^t$)- |
| ″ | ″ | pMz-Tyr($Bu^t$)- | pMz-Tyr($Bu^t$)- |
| ″ | ″ | Z-Asp(OBzl)- | Z-Asp(OBzl)- |
| ″ | ″ | Boc-Asp($OBu^t$)- | Boc-Asp($OBu^t$)- |
| ″ | ″ | pMz-Asp($OBu^t$)- | pMz-Asp($OBu^t$)- |
| ″ | ″ | Z-Ala- | Z-Ala- |
| ″ | ″ | Boc-Ala- | Boc-Ala- |
| ″ | ″ | pMz-Ala | pMz-Ala |
| ″ | ″ | Z-Ser(Bzl)- | Z-Ser(Bzl)- |
| ″ | ″ | Boc-Ser($Bu^t$)- | Boc-Ser($Bu^t$)- |
| ″ | ″ | pMz-Ser($Bu^t$)- | pMz-Ser($Bu^t$)- |

Table 2 (cont'd)

| X | $R_3$ | $R_4$ | $R_5$ |
|---|---|---|---|
| 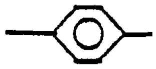 | $-CH_2OH$ | Z-Pro- | Z-Pro- |
| " | " | Boc-Pro- | Boc-Pro- |
| " | " | pMz-Pro- | pMz-Pro- |
| " | " | Z-Gln- | Z-Gln- |
| " | " | Boc-Gln- | Boc-Gln- |
| " | " | pMz-Gln- | pMz-Gln- |
| " | " | Z-Lys(Z)- | Z-Lys(Z)- |
| " | " | Boc-Lys(Boc)- | Boc-Lys(Boc)- |
| " | " | pMz-Lys(pMz)- | pMz-Lys(pMz)- |
| " | " | Z-Leu-Leu- | Z-Leu-Leu- |
| " | " | Boc-Leu-Leu- | Boc-Leu-Leu- |
| " | " | pMz-Leu-Leu- | pMz-Leu-Lue- |
| " | " | Z-β-Ala- | Z-β-Ala- |
| " | " | Boc-β-Ala- | Boc-β-Ala- |
| " | " | pMz-β-Ala- | pMz-β-Ala |
| " | " | Z-AHPA- | Z-AHPA- |
| " | " | Boc-AHPA- | Boc-AHPA- |
| " | " | pMz-AHPA- | pMz-AHPA- |
| " | H | Z-Leu- | Z-Leu- |
| " | " | Boc-Leu- | Boc-Leu- |
| " | " | pMz-Leu- | pMz-Leu- |
| " | " | Z-Leu-Leu- | Z-Leu-Leu- |
| " | " | Boc-Leu-Leu- | Boc-Leu-Leu- |
| " | " | pMz-Leu-Leu- | pMz-Leu-Leu- |

Table 2 (cont'd)

| X | $R_3$ | $R_4$ | $R_5$ |
|---|---|---|---|
| 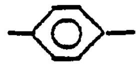 | H | Z-Phe- | Z-Phe- |
| 〃 | 〃 | Boc-Phe- | Boc-Phe- |
| 〃 | 〃 | pMz-Phe- | pMz-Phe- |
| $-(CH_2)_3$ | $-CH_2OH$ | L-Leu- | L-Leu- |
| 〃 | 〃 | Boc-Leu- | Boc-Leu- |
| 〃 | 〃 | pMz-Leu- | pMz-Leu- |
| 〃 | 〃 | Z-Gly- | Z-Gly- |
| 〃 | 〃 | Boc-Gly- | Boc-Gly- |
| 〃 | 〃 | pMz-Gly- | pMz-Gly- |
| 〃 | 〃 | Z-Leu-Leu- | Z-Leu-Leu- |
| 〃 | 〃 | Boc-Leu-Leu- | Boc-Leu-Leu- |
| 〃 | 〃 | pMz-Leu-Leu- | pMz-Leu-Leu- |
| 〃 | H | Z-Leu-Leu- | Z-Leu-Leu- |
| 〃 | 〃 | Boc-Leu-Leu- | Boc-Leu-Leu- |
| 〃 | 〃 | pMz-Leu-Leu- | pMz-Leu-Leu- |
| 〃 | 〃 | Z-Leu- | Z-Leu- |
| 〃 | 〃 | Boc-Leu- | Boc-Leu- |
| 〃 | 〃 | pMz-Leu- | pMz-Leu- |
| 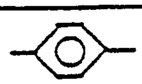 | $-CH_2OBzl$ | Z-Leu- | Z-Leu- |
| 〃 | $-CH_2OBu^t$ | Boc-Leu- | Boc-Leu- |
| 〃 | 〃 | pMz-Leu- | pMz-Leu- |
| 〃 | $-CH_2OBzl$ | Z-Leu-Leu- | Z-Leu-Leu- |
| 〃 | $-CH_2OBu^t$ | Boc-Leu-Leu- | Boc-Leu-Leu- |
| 〃 | 〃 | pMz-Leu-Leu- | pMz-Leu-Leu- |

When the compounds of the present invention prepared as described above are used as medicines for manmarian having immuno-deficiency, they are formulated into preparations with, if necessary, excipients in an ordinary manner and administered orally or parenterally.

When they are used as an injection, the amount of the active ingredient is adjusted usually in the range of 0.1 to 30 wt.%, preferably 1 to 10 wt.%. When they are administered orally, they are used in the form of tablet, capsule, powder, granule, liquid or dry syrup. The capsule, granule and powder contain usually 5 to 100 wt.%, preferably 25 to 100 wt.%, of the active ingredient.

The dose is determined suitably depending on the age, body weight and symptoms of the patient and the therapeutic purpose. The therapeutic dose is usually 1 to 100 mg/kg/day in the parenteral administration

and 5 to 500 mg/kg/day in the oral administration.

[Effects]

The physiological activities of the compounds of the present invention will be shown by the following experimental examples.

1. Experimental method:

(a) Antibody production-enhancing effect:

Female $CDF_1$-SLC mice (each group consisting of 5 mice) were sensitized i.v. with 1 x $10^8$/0.2 mℓ of sheep red blood cells (SRBC). A compound of the present invention was diluted to various concentrations with physiological saline and administered in an amount of 0.1 mℓ for 10 g of the body weight once a day (0.1 mg/l0 g/day) repeatedly for 3 days starting from one day after the sensitization. Only the physiological saline was administered to a control group.

Four days after the sensitization, the mice were sacrificed and the number of anti-SRBC antibody-producing cells (plague-forming cells, PFC) in splenocytes were counted to calculate the number of PFC for $10^6$ splenocytes. The effect of the compound of the present invention was represented by an enhancement rate (%) of the number of PFC in the group to which the compound of the present invention was administered as compared with the number of PFC in the control group.

$$\text{Enhancement rate} = \left(\frac{\text{No. of PFC in the test group}}{\text{No. of PFC in the control group}} - 1\right) \times 100$$

2. Experimental results:

The antibody production-enhancing effects of typical examples of the compounds of the present invention are shown in Table 3. The physiological saline was used as the control.

Table 4

| Antibody production-enhancing effect of the compound of the present invention | |
|---|---|
| Chemical | Antibody production-enhancement rate (%) (dose: 6.25 mg/kg) |
| Compound No. 1 | 95 |
| Physiological saline | 0 |

It is apparent from the above-described test examples that the compounds of the present invention have an excellent immune-enhancing effect and the utilization thereof as therapeutic and preventive agents for various infections diseases and as antineoplastic agents is expected.

The following examples will further illustrate the present invention. In the following examples, Rf values of thin layer chromatography (TLC) were determined by development to a height of about 8 cm with Silica Gel 60 $F_{254}$ plate having a thickness of 0.25 mm (a product of Merck) with a developing solvent which will be mentioned below and dividing the distance from the origin to the center of the spot of the intended product by the distance from the origin to the front of the developing solvent. The detection was conducted

by UV spectrophotometry (2537 Å) with ninhydrin and Sakaguchi's reagent.

Example 1

10-{N-[4-(4-Guanidinophenyl)butyryl]-L-seryl}-1,5-di-L-leucyl-1,5,10-triazadecane trihydrochloride (compound No. 1):

5.92 g (5.24 mmol) of oily 10-{N-[4-(4-guanidinophenyl)butyryl]-O-benzyl-L-seryl}-1,5-di-(N-benzyloxycarbonyl-L-leucyl)-1,5,10-triazadecane hydrochloride was dissolved in a mixture of 50 mℓ of methanol with 30 mℓ of acetic acid. 0.6 g of palladium black was added to the solution and the mixture was heated at 55°C to conduct catalytic reduction under atmospheric pressure for 6 h. After the completion of the reaction, the catalyst was removed by filtration and the filtrate was concentrated under reduced pressure. The oily residue was suspended in 50 mℓ of acetone and the supernatant liquid was decanted twice.

After concentration under reduced pressure, 4.8 g of white crystals were obtained and dissolved in 70 mℓ of distilled water. The solution was passed through a column packed with 500 mℓ of Cm-Sephadex® C-25 ($Na^+$) and eluted by gradient elution between 2.5 ℓ of distilled water and 2.5 ℓ of a 1.3 M aqueous sodium chloride solution. Fractions containing the intended compound were collected and concentrated to dryness under reduced pressure. Methanol was added to the dry product and precipitated sodium chloride was removed by filtration. The intended compound was separated from the oily product and purified as follows: to remove a small amount of sodium chloride remaining therein, the resulting oily product was dissolved in 30 mℓ of methanol and the solution was passed through a column packed with 210 mℓ of Sephadex® LH-20. After elution with methanol, fractions containing the intended compound were collected and concentrated under reduced pressure. To remove a small amount of impurities, the oily product thus obtained was dissolved in 20 mℓ of distilled water and the solution was passed through a column packed with 180 mℓ of HP-20® (Mitsubishi Chemical Industries, Ltd.). After washing with 270 mℓ of distilled water, the elution was conducted with 20 to 30% methanol. Fractions containing the intended compound were collected and concentrated under reduced pressure. The oily product thus obtained was dissolved in 12 mℓ of distilled water. An insoluble matter was removed by filtration and the filtrate was freeze-dried to give 1.73 g (yield: 37.4%) of the intended compound.

N M R ( $D_2O$ , external T M S )
$\delta$ = 1.2 ~ 1.8 ( bd, 1 2 H, J=5Hz), 1.8~3.4 (m, 1 8 H ), 3.4~4.1 (m, 8 H ), 4.1~4.4 ( d, 2 H, J= 6 Hz ), 4.2~5.0 ( m, 3 H ), 7.5~8.0 ( m, 4 H )
I R ( K Br )
$\nu$ ( $cm^{-1}$ ) =3240, 2940, 1635, 1510, 1460, 1365, 1255, 1170, 1125, 1060.

TLC (chloroform/methanol/17% aqueous ammonia = 6 : 2.5 : 0.5 v/v)
Rf = 0.26
$[\alpha]_D^{20}$ - 4.9° (C = 1.0, $H_2O$)

The compounds of the general formula [I] can be prepared by treating the compounds of the general formula [II] in the same manner as that of Example 1.

When the benzyloxycarbonyl group which is a protective group for the amino group of an amino acid in position 1 of the compound of the general formula [II] is replaced with a tert-butyloxycarbonyl, p-methoxybenzyloxycarbonyl or t-amyloxycarbonyl group and the protective group for the carboxyl or hydroxyl group is a tert-butyl group, the intended compound can be prepared by treating the compound with trifluoroacetic acid instead of the catalytic reduction and then treating the product in the same manner as that of Example 1. When the compound of the general formula [II] has both benzyloxycarbonyl and tert-butyl groups as the protective groups, it is catalytically reduced at first, then treated with trifluoroacetic acid and finally treated in the same manner as that of Example 1 to give the intended compound.

The compounds of the general formula [I] were prepared from those of the general formula [II] as shown in the table given below.

The compounds of the general formula [II] were prepared as shown in the Referential Examples given below or in the same manner as that shown therein.

Referential Example 1

Preparation of 10-{N-[4-(4-Guanidinophenyl)butyryl]-O-benzyl-L-seryl}-1,5-di-(N-benzyloxycarbonyl-L-leucyl)-1,5,10-triazadecane hydrochloride:

(1) 10-tert-Butyloxycarbonyl-1,5,10-triazadecane:

18.9 g (100 mmol) of mono-N-tert-butyloxy carbonyl-1,4-butanediamine (see Japanese Patent Laid-Open No. 57-192347) was dissolved in 150 mℓ of chloroform. 5.57 g (105 mmol) of acrylonitrile was added to the solution under cooling with ice and a reaction was conducted at room temperature for 3 days. The reaction mixture was concentrated under reduced pressure to give 23.4 g of an oily substance.

23.4 g of the oily substance was dissolved in 260 mℓ of ethanol saturated with ammonia. 20 g of Raney nickel was added to the solution and hydrogenation was conducted at room temperature under 60 atm for 5 h. After the completion of the reaction, the catalyst was removed by filtration and the filtrate was concentrated under reduced pressure to give 23.7 g (yield: 96.7%) of the intended compound.

NMR ($D_2O$, external TMS)
$\delta$ = 1.6 ~ 2.5 (m, 6H), 1.9 (s, 9H), 2.7 ~ 3.5 (m, 6H), 3.4 ~ 3.8 (m, 3H).

(2) 10-tert-Butyloxycarbonyl-1,5-di-(N-benzyloxycarbonyl-L-leucyl)-1,5-10-triazadecane:

4.0 g (16.30 mmol) of oily 10-tert-butyloxy carbonyl-1,5,10-triazadecane and 8.65 g (32.6 mmol) of N-benzyloxycarbonyl-L-leucine were dissolved in 50 mℓ of dichloromethane. 6.87 g (35.86 mmol) of 1-ethyl-3-(3′-dimethylaminopropyl)carbodiimide hydrochloride was added to the solution under cooling with ice and a reaction was conducted at room temperature overnight. The reaction mixture was concentrated under reduced pressure to give an oily substance, which was dissolved in 200 mℓ of ethyl acetate and washed with a 5% aqueous sodium carbonate solution and then with a saturated aqueous common salt solution. The organic layer was dried over anhydrous sodium sulfate and concentrated under reduced pressure to give 10.0 g of an oily substance.

The oily substance thus obtained was subjected to column chromatography with Silica Gel 60 (Merck) and developed with a mixture of chloroform and acetone (10:1, v/v) to give 6.9 g (yield: 57.2%) of an oily substance.

N M R ( $CDCl_3$ )
$\delta$ =0.5~1.1 ( d, 1 2 H, J=5 Hz ), 1.1~2.1 ( b, 12H ) 1.4 ( s, 9 H ), 2.6~3.9 ( b, 8 H ), 3.9~5.4 ( b, 3 H ), 5.1 ( s, 4 H ), 5.4~6.1 ( b, 2 H ), 6.4~8.1 ( b, H ), 7.34 ( s, 1 0 H )

TLC (chloroform/acetone = 10:1 v/v)
Rf = 0.16

(3) 1,5-Di-(N-benzyloxycarbonyl-L-leucyl)-1,5,10-triazadecane hydrochloride:

5.4 g (7.3 mmol) of 10-tert-butyloxycarbonyl-1,5-di-(N-benzyloxycarbonyl-L-leucyl)-1,5,10-triazadecane was dissolved in dichloromethane. 10 mℓ of 4N hydrochloric acid in dioxane was added to the solution under cooling with ice and a reaction was conducted at room temperature for 2 h. The reaction mixture was concentrated under reduced pressure and an oily substance thus obtained was suspended in 50 mℓ of n-hexane. The supernatant was decanted twice and the residue was concentrated under reduced pressure to give 5.1 g (quantitative) of the oily intended compound.
TLC (chloroform/methanol = 10:1, v/v)
Rf = 0.2.

(4) 10-(N-tert-Dutyloxycarbonyl-O-benzyl-L-seryl)-1,5-di-(N-benzyloxycarbonyl-L-leucyl)-1,5,10-triazadecane:

4.5 g (6.09 mmol) of 1,5-di-(N-benzyloxycarbonyl-L-leucyl)-1,5,10-triazadecane hydrochloride was dissolved in 50 mℓ of dichloromethane. 1.0 g (10.05 mmol) of triethylamine was added to the solution under cooling with ice. 2.39 g (6.09 mmol) of N-tert-butyloxycarbonyl-O-benzyl-L-serine N-hydroxysuccinimide ester was added thereto and a reaction was conducted at room temperature overnight. The reaction mixture was concentrated under reduced pressure and the residue was dissolved in 200 mℓ of ethyl acetate. The solution was washed with 5% phosphoric acid, a 5% aqueous sodium carbonate solution and a saturated aqueous common salt solution, successively. The organic layer was dried over anhydrous sodium sulfate. The filtrate was concentrated under reduced pressure to give 10 g of a pale yellow oily substance, which was subjected to column chromatography with Silica Gel 60 (Merck) and developed with a mixture of chloroform and acetone (10:1, v/v) and then with a mixture of chloroform and methanol (50:1, v/v) to give 5.0 g (yield: 86.2%) of the intended compound in the form of a pale yellow oil.

N M R ( $CDCl_3$ )
δ=0.6~1.1 ( d, 1 2 H, J=5 Hz ), 1.1~2.1 ( b,12H), 1.4 ( s, 9 H ), 2.5~4.0 ( b, 1 0 H ), 4.0~8.0 ( b, 6 H ), 4.5 ( s, 2 H ), 5.0 ( s, 4 H ), 5.3~5.9 ( b, 2 H ), 7.3 ( s, 1 5 H )

TLC (chloroform/methanol = 40:1 v/v)
Rf = 0.28

(5) 10-(O-Benzyl-L-seryl)-1,5-di-(N-benzyloxycarbonyl-L-leucyl)-1,5,10-triazadecane hydrochloride:

5.0 g (5.24 mmol) of 10-(N-tert-butyloxycarbonyl-O-benzyl-L-seryl)-1,5-di-(N-benzyloxycarbonyl-L-leucyl)-1,5,10-triazadecane was dissolved in 5 mℓ of dichloromethane. 10 mℓ of 4N-hydrochloric acid in dioxane was added to the solution under cooling with ice and a reaction was conducted for 2 h. The reaction mixture was concentrated under reduced pressure. An oily product thus obtained was suspended in 50 mℓ of n-hexane and the supernatant liquid was decanted. This operation was repeated twice and the residue was concentrated under reduced pressure to give 4.8 g (quantitative) of the intended compound in the form of white crystals.
TLC (chloroform/methanol = 10:1, v/v)
Rf = 0.42

(6) 10-{N-4-(4-Guanidinophenyl)butyryl]-O-benzyl-L-seryl}-1,5-di-(N-benzyloxycarbonyl-L-leucyl)- 1,5,10-triazadecane hydrochloride:

1.49 g (5.78 mmol) of 4-(4-guanidinophenyl)butyric acid hydrochloride was dissolved in 20 mℓ of dimethylformamide. 0.79 g (6.9 mmol) of N-hydroxysuccinimide and 1.43 g (6.9 mmol) of N,N′-dicyclohexylcarbodiimide were added to the solution and a reaction was conducted at room temperature overnight. N,N′-Dicyclohexylurea thus precipitated was separated by filtration and the filtrate was used as it was in the next step.

4.8 g (5.24 mmol) of 10-(O-benzyl-L-seryl)-1,5-di-(N-benzyloxycarbonyl-L-leucyl)-1,5,10-triazadecane hydrochloride was dissolved in 40 mℓ of dimethylformamide. 0.64 g (6.3 mmol) of triethylamine was added to the solution under cooling with ice. Then the above-mentioned solution of 4-(4-guanidinophenyl)butyric acid hydrochloride N-hydroxysuccinimide ester in dimethylformamide was added thereto and a reaction was conducted at room temperature for 5 h. The reaction mixture was concentrated under reduced pressure and an oily residue thus obtained was dissolved in 200 mℓ of dichloromethane. The solution was washed with a saturated aqueous common salt solution thrice. The organic layer was dried over anhydrous sodium sulfate. The filtrate was concentrated under reduced pressure to give 6.4 g (quantitative) of the intended compound in the form of a pale yellow oil.
NMR ($CD_3OD$)

δ=0.6~1.1 ( d, 1 2 H, J=5 Hz ), 1.1~3.9 ( m, 28H), 3.9~4.9 ( m, 3 H ), 3.9~7.6 ( b, 9 H ), 4.5 1 ( s, 2 H ), 5.0 ( s, 4 H ), 6.8~7.4 ( m, 4 H ), 7.3 ( s, 1 5 H ).
I R ( K B r )
ν ($cm^{-1}$) = 3260, 3030, 2930, 1630, 1525, 1445, 1245, 1100, 1035.

TLC (chloroform/methanol/17% aqueous ammonia = 6:1.5:0.25 v/v)

Rf = 0.28

Compounds of the general formula [II] which were the starting compounds for the compound Nos. 2 to 11 were prepared in the same manner as that described above.

| Example | Compound of general formula [II] | Compound of general formula [I] |
| --- | --- | --- |
| 2 | 10-{N-[4-(4-Guanidinophenyl)butyryl]-0-benzyl-L-seryl}-1,5-(di--N-benzyloxycarbonyl-L-phenylalanyl-1,5,10-triazadecane hydrochloride<br>NMR ($CD_3OD$) $\delta$ = 1.2 ~ 3.4 (m, 24H), 3.5 ~ 3.6 (d, J = 5 Hz, 2 H), 4.1 ~ 5.0 (m, 5H), 5.0 (s, 4H), 3.4 ~ 7.4 (m, 9H), 6.9 ~ 7.1 (m, 4H), 7.2 ( , 25H)<br>IR (KBr) $\nu(cm^{-1})$ = 3275, 3050, 2930, 1615, 1501, 1420, 1218, 1080, 1020, 1010.<br>TLC (Chloroform/methanol/17% aqueous ammonia = 6:1.5:0.25 v/v.)<br>Rf = 0.34 | 10-{N-[4-(4-Guanidinophenyl)butyryl]-L-seryl}-1,5-di-L-phenylalanyl-1,5,10-triazadecane trihydrochloride (Compound No. 2)<br>NMR ($D_2O$, external TMS) $\delta$ = 1.4 ~ 3.7 (m, 24H), 4.3 ~ 4.4 (d, J = 5 Hz, 2H), 4.4 ~ 5.1 (m, 3H), 7.5 ~ 7.8 (m, 4H), 7.7 (s, 10H)<br>IR (KBr) $\nu(cm^{-1})$ = 3225, 3050, 2925, 2310, 1625, 1510, 1450, 1362, 1250, 1070.<br>TLC (Chloroform/methanol/17% aqueous ammonia = 6:25:0.5 v/v)<br>Rf = 0.24<br>$[\alpha]_D^{20}$ + 19.6° (C = 0.98 $H_2O$) |

| Example | Compound of general formula [II] | Compound of general formula [I] |
|---|---|---|
| 3 | 10-{N-[4-(4-Guanidinophenyl)butyryl]-0-benzyl-L-seryl}-1,5--(di-N-benzyloxycarbonyl-L-tyrosyl-1,5,10-triazadecane hydrochloride<br>NMR ($CD_3OD$) $\delta$ = 0.9 ~ 3.5 (m, 26H), 3.5 ~ 3.9 (bd, 2H, J = 5 Hz), 3.9 ~ 8.5 (b, 9H), 4.0 ~ 5.1 (m, 3H), 4.48 (s, 2H), 5.0 (s, 4H), 6.4 ~ 7.3 q, 8H, J = 8.5 Hz), 6.9 ~ 7.5 (m, 4H), 7.25 (s, 15H).<br>IR (KBr) $\nu$($cm^{-1}$) = 3290, 2940, 1640, 1515, 1445, 1235.<br>TLC (Chloroform/methanol/17% aqueous ammonia = 6:2.5:0.5 v/v)<br>Rf = 0.38 | 10-{N-[4-(4-Guanidinophenyl)butyryl]-L-seryl}-1,5-di-L-tyrosyl-1,5,10-triazadecane trihydrochloride (Compound No. 3)<br><br>NMR ($D_2O$, external TMS) $\delta$ = 1.4 ~ 4.0 (m, 24H), 4.1 ~ 4.4 (d, 2H, J = 5 Hz), 4.4 ~ 5.1 (m, 3H), 7.1 ~ 7.8 (q, 8H, J = 8.5 Hz), 7.4 ~ 7.9 (m, H).<br><br>IR (KBr) $\nu$($cm^{-1}$) = 3240, 2940, 1635, 1510, 1365, 1230.<br>TLC (Chloroform/methanol/17% aqueous ammonia = 6:4:1 v/v)<br><br>Rf = 0.12<br>$[\alpha]_D^{20}$ + 22.7° (C = 1.0, $H_2O$) |

| Example | Compound of general formula [II] | Compound of general formula [I] |
|---|---|---|
| 4 | 10-{N-[4-(4-Guanidinophenyl)butyryl]-0-benzyl-L-seryl}-1,5-(di-N--benzyloxycarbonyl-$\beta$-O-benzyl-L-aspartyl)-1,5,10-triazadecane hydrochloride<br>NMR ($CD_3OD$) $\delta$ = 1.1 ~ 3.7 (m, 24H), 3.5 ~ 3.8 (d, 2H, J = 5 Hz), 4.47 (s, 2H), 4.0 ~ 8.9 (b, 12H), 5.03 (s, 8H), 6.9 ~ 7.5 (m, 4H), 7.27 (s, 25H).<br>IR (KBr) $\nu(cm^{-1})$ = 3310, 2940, 1710, 1640, 1530, 1515, 1450, 1260, 1045.<br>TLC (Chloroform/methanol/17% aqueous ammonia = 6:1.5:0.25 v/v)<br>Rf = 0.30 | 10-{N-[4-(4-Guanidinophenyl)butyryl]-L-seryl}-1,5-di-L-aspartyl-1,5,10-triazadecane trihydrochloride (Compound No. 4)<br><br>NMR ($D_2O$, external TMS) $\delta$ = 1.6 ~ 4.3 (m, 24H), 4.1 ~ 4.5 (d, 2H, J = 5 Hz), 4.6 ~ 5.0 (t, H, J = 5 Hz), 4.9 ~ 5.4 (m, 2H), 7.5 ~ 8.1 (m, 4H)<br><br>IR (KBr) $\nu(cm^{-1})$ = 3380, 2940, 1710, 1640, 1510, 1440, 1410, 1250.<br><br>TLC (Chloroform/methanol/17% aqueous ammonia = 6:4:1 v/v)<br><br>Rf = 0.11<br>$[\alpha]_D^{20}$ - 11.2° (C = 1.0, $H_2O$) |

| Example | Compound of general formula [II] | Compound of general formula [I] |
|---|---|---|
| 5 | 10-{N-[4-(4-Guanidinophenyl)butyryl]-0-benzyl-L-seryl}-1,5--(di-N-benzyloxycarbonyl-L-alanyl)-1,5,10-triazadecane hydrochloride<br>NMR ($CD_3OD$) $\delta$ = 1.0 ~ 3.4 (m, 26H), 3.4 ~ 3.7 (d, J = 5 Hz, 2H), 3.7 ~ 4.1 (m, 3H), 4.3 (s, 2H), 4.9 (s, 4H), 3.5 ~7.5 (m, 9H), 6.8 ~ 7.3 (m, 4H), 7.1 (s, 15H).<br>IR (KBr) $\nu$(cm$^{-1}$) = 3280, 2930, 1700, 1635, 1510, 1445, 1243, 1095, 1060, 1020.<br>TLC (Chloroform/methanol/17% aqueous ammonia = 6:1.5:0.25 v/v)<br>Rf = 0.47 | 10-{N-[4-(4-Guanidinophenyl)butyryl]-L-seryl}-1,5-di-L-alanyl-1,5,10-triazadecane trihydrochloride (Compound No. 5)<br>NMR ($D_2O$, external TMS) $\delta$ = 1.8 ~ 2.1, (d, J = 7 Hz, 3H), 1.9 ~ 2.2 (d, J = 7 Hz, 3H), 2.2 ~ 4.1 (m, 20H), 4.1 ~ 4.4 (d, J = 5 Hz, 2H), 4.4 ~5.0 (m, 3H), 7.5 ~ 8.0 (m, 4H).<br>IR (KBr) $\nu$(cm$^{-1}$) = 3300, 3000, 1670, 1645, 1420, 1360, 1240, 1200, 1100.<br>TLC (Chloroform/methanol/17% aqueous ammonia = 2:1:1 v/v)<br>Rf = 0.40<br>$[\alpha]_D^{20}$ - 10.6° (C = 0.97, $H_2O$) |

| Example | Compound of general formula [II] | Compound of general formula [I] |
|---|---|---|
| 6 | 10-{N-[4-(4-Guanidinophenyl)butyryl]-0-benzyl-L-seryl}-1,5-(di--N-benzyloxycarbonyl-O-benzyl-L-seryl)-1,5,10-triazadecane hydrochloride<br>NMR ($CD_3OD$) δ = 1.2 ~ 3.5 (m, 20H), 3.5 ~ 3.8 (d, J = 5 Hz, 6H), 4.1 ~ 4.6 (m, 3H), 4.4 (s, 6H), 4.9 ~ 5.1 (s, 4H), 4.1 ~ 7.5 (m, 9H), 6.9 d1>² 7.5 (m, 4H), 7.2 (s, 25H).<br>IR (KBr) $\nu(cm^{-1})$ = 3300, 3050, 2925, 2850, 1710, 1615, 1510, 1425, 1360, 1240, 1100, 1020.<br>TLC (Chloroform/methanol/17% aqueous ammonia = 6:1.5:0.25 v/v)<br>Rf = 0.41 | 10-{N-[4-(4-Guanidinophenyl)butyryl]-L-seryl}-1,5-di-L-seryl-1,5,10-triazadecane trihydrochloride (Compound No. 6)<br><br>NMR ($D_2O$, external TMS) δ = 1.5 ~ 4.1 (m, 20H), 4.1 ~ 4.6 (m, 6H), 4.6 ~ 5.2 (m, 3H), 7.5 ~ 8.0 (m, 4H).<br><br>IR (KBr) $\nu(cm^{-1})$ = 3300, 2925; 1635, 1500, 1360, 1250, 1050.<br><br>TLC (Propanol/pyridine/water/acetic acid = 6:4:3:2 v/v)<br><br>Rf = 0.36<br>$[\alpha]_D^{20}$ - 9.6° (C = 0.98, $D_2O$) |

| Example | Compound of general formula [II] | Compound of general formula [I] |
|---|---|---|
| 7 | 10-{N-[4-(4-Guanidinophenyl)butyryl]-0-t-butyl-L-seryl}-1,-5-(di-N-t-butoxycarbonyl-L-prolyl)-1,5,10-triazadecane hydrochloride<br>NMR ($CD_3OD$) $\delta$ = 0.9 ~ 4.0 (m, 32H), 1.3 (s, 9H), 1.5 (s, 18H), 4.0 ~ 4.7 (m, 3H), 4.0 ~ 7.6 (m, 9H), 7.0 ~ 7.6 (m, 4H).<br>IR (KBr) $\nu(cm^{-1})$ = 3300, 3010, 1690, 1570, 1540, 1500, 1430, 1400, 1280, 1190, 1145, 1100.<br>TLC (Chloroform/methanol/17% aqueous ammonia = 6:1.5:0.25 v/v)<br>Rf = 0.34 | 10-{N-[4-(4-Guanidinophenyl)butyryl]-L-seryl}-1,5-di-L-prolyl-1,5,10-triazadecane trihydrochloride (Compound No. 7)<br>NMR ($D_2O$, external TMS) $\delta$ = 1.8 ~ 4.2 (m, 32H), 4.2 ~ 4.5 (d, J = 5 Hz, 2H), 4.6 ~ 5.2 (m, 3H), 7.6 ~ 8.0 (m, 4H).<br>IR (KBr) $\nu(cm^{-1})$ = 3300, 3075, 2930, 1660, 1640, 1550, 1510, 1440, 1370, 1250.<br>TLC (Propanol/pyridine/water/acetic acid = 6:4:3:2 v/v)<br>Rf = 0.43<br>$[\alpha]_D^{20}$ -51.8° (C = 1.0, $H_2O$) |

| Example | Compound of general formula [II] | Compound of general formula [I] |
|---|---|---|
| 8 | 10-{N-[4-(4-Guanidinophenyl)butyryl]-0-benzyl-L-seryl}-1,5--(di-N-benzyloxycarbonyl-β-alanyl)-1,5,10-triazadecane hydrochloride<br>NMR ($CD_3OD$) δ = 0.9 ~ 3.8 (m, 38H), 1.1 ~ 1.4 (d, J = 7Hz, 6H), 4.5 (s, 2H), 4.4 ~ 4.7 (m, 1H), 5.0 (s, 4H), 3.6 ~ 7.6 (m, 9H), 6.9 ~ 7.4 (m, 4H), 7.2 (s, 15H).<br>IR (KBr) $\nu(cm^{-1})$ = 3275, 2925, 2850, 1700, 1610, 1500, 1440, 1360, 1240, 1100, 1060.<br>TLC (Chloroform/methanol/17% aqueous ammonia = 6:1.5:0.25 v/v)<br>Rf = 0.47 | 10-{N-[4-(4-Guanidinophenyl)butyryl]-L-seryl}-1,5-di-β-analyl-1,5,10-triazadecane trihydrochloride (Compound No. 8)<br><br>NMR ($D_2O$, external TMS) δ = 1.7 ~ 4.1 (m, 32H), 4.1 ~ 4.4 (d, J = 5 Hz, 2H), 4.6 ~ 5.0 (m, 1H), 7.5 ~ 8.1 (m, 4H).<br><br>IR (KBr) $\nu(cm^{-1})$ = 3375, 2940, 1615, 1545, 1510, 1455, 1380, 1315, 1250, 1200, 1090.<br>TLC (Propanol/pyridine/water/acetic acid = 6:4:3:2 v/v)<br><br>Rf = 0.24<br>$[\alpha]_D^{20}$ - 10.1° (C = 1.0, $H_2O$) |

| Example | Compound of general formula [II] | Compound of general formula [I] |
|---|---|---|
| 9 | 10-{N-[4-(4-Guanidinophenyl)butyryl]-0-benzyl-L-seryl}-1,5--(di-N-benzyloxycarbonyl-L-glycyl)-1,5,10-triazadecane hydrochloride<br>NMR ($CD_3OD$) $\delta$ = 0.8 ~ 3.9 (m, 24H), 3.9 ~ 4.1 (m, 2H), 4.3 ~ 4.6 (m, 3H), 5.0 (s, 4H), 4.2 ~ 7.5 (m, 9H), 7.0 ~ 7.5 (m, 4H), 7.2 (s, 15H).<br>IR (KBr) $\nu(cm^{-1})$ = 3300, 3070, 2900, 1605, 1520, 1430, 1225, 1105, 1050.<br>TLC (Chloroform/methanol/17% aqueous ammonia = 6:1.5:0.25 v/v)<br>Rf = 0.42 | 10-{N-[4-(4-Guanidinophenyl)butyryl]-L-seryl}-1,5-di-L-glycyl-1,5,10-triazadecane trihydrochloride (Compound No. 9)<br><br>NMR ($D_2O$, external TMS) $\delta$ = 1.7 ~ 4.1 (m, 24H), 4.1 ~ 4.4 (d, J = 6 Hz, 4H), 4.4 ~ 4.7 (d, J = 5 Hz, 2H), 4.7 ~ 5.0 (m, 1H), 7.6 ~ 8.0 (m, 4 ).<br><br>IR (KBr) $\nu(cm^{-1})$ = 3250, 3075, 2930, 1642, 1555, 1518, 1503, 1415, 1360, 1250, 1212, 1070.<br>TLC (Chloroform/methanol/17% aqueous ammonia = 2:1:1 v/v)<br><br>Rf = 0.26<br>$[\alpha]_D^{20}$ - 19.4° (C = 1.0, $H_2O$) |

| Example | Compound of general formula [II] | Compound of general formula [I] |
| --- | --- | --- |
| 10 | 10-{N-[4-(4-Guanidinophenyl)butyryl]-0-benzyl-L-seryl}-1,5-(-di-N-benzyloxycarbonyl-L-glutaminyl)-1,5,10-triazadecane hydrochloride<br>NMR ($CD_3OD$) $\delta$ = 1.3 ~ 5.2 (m, 30H), 3.7 ~ 3.9 (m, 2H), 3.9 ~ 4.3 (m, 1H), 4.5 (s, 2H), 5.1 (s, 4H), 3.5 ~ 7.5 (m, 13H), 6.9 ~ 7.5 (m, 4H), 7. (s, 15H).<br>IR (KBr) $\nu(cm^{-1})$ = 3300, 2920, 2880, 1640, 1550, 1440, 1250, 1200, 1125, 1045.<br>TCLC (Chloroform/methanol/17% aqueous ammonia = 6:1.5:0.25 v/v)<br>Rf = 0.19 | 10-{N-[4-(4-Guanidinophenyl)butyryl]-L-seryl}-1,5-di-L-glutaminyl-1,5,10-triazadecane trihydrochloride (Compound No. 10)<br><br>NMR ($D_2O$, external TMS) $\delta$ = 1.6 ~ 4.1 (m, 28H), 4.1 ~ 4.4 (d, J = 5 Hz, 2H), 4.5 ~ 4.9 (m, 3H), 7.5 ~ 7.9 (m, 4H).<br><br>IR (KBr) $\nu(cm^{-1})$ = 3230, 2930, 2460, 1640, 1540, 1520, 1440, 1370, 1250, 1200, 1150, 1060, 1030.<br>TLC (Chloroform/methanol/17% aqueous ammonia = 2:1:1 v/v)<br><br>Rf = 0.30<br>$[\alpha]_D^{20}$ - 17.5° (C = 1.04, $H_2O$) |

| Example | Compound of general formula [II] | Compound of general formula [I] |
|---|---|---|
| 11 | 10-{N-[4-(4-Guanidinophenyl)butyryl]-0-benzyl-L-seryl}-1,-5-(di-N-benzyloxycarbonyl-L-lysyl)-1,5,10-triazadecane hydrochloride<br>NMR ($CD_3OD$) $\delta$ = 1.0 ~ 3.5 (m, 26H), 3.5 ~ 3.8, (d, J = 5 Hz, 2H), 4.5 (s, 2H), 5.0 (s, 4H), 3.5 ~ 5.1 (m, 6H), 3.5 ~ 7.4 (m, 13H), 6.9 ~ 7.4, (m, 4H), 7.3 (s, 15H).<br>IR (KBr) $\nu(cm^{-1})$ = 3300, 3060, 2930, 2850, 1680, 1630, 1520, 1445, 1224, 1100, 1020, 1010.<br>TLC (Chloroform/methanol = 10:1 v/v)<br>Rf = 0.18 | 10-{N-[4-(4-Guanidinophenyl)butyryl]-L-seryl}-1,5-di-L-lysyl-1,5,10-triazadecane trihydrochloride (Compound No. 11)<br><br>NMR ($D_2O$, external TMS) $\delta$ = 1.7 ~ 3.8 (m, 32H), 4.1 ~ 4.3 (d, J = 5 Hz, 2H), 4.1 ~ 5.1 (m, 7H), 7.5 ~ 7.9 (m, 4H).<br><br>IR (KBr) $\nu(cm^{-1})$ = 3380, 3050, 2940, 1990, 1615, 1505, 1370, 1250, 1115, 1060.<br><br>TLC (n-Propanol/pyridine/water/acetic acid = 6:4:3:2 v/v)<br>Rf = 0.14<br>$[\alpha]_D^{20}$ + 21.7° (C = 0.97, $H_2O$) |

## Claims

1. New spergualin-related compounds of the following general formula [I]:

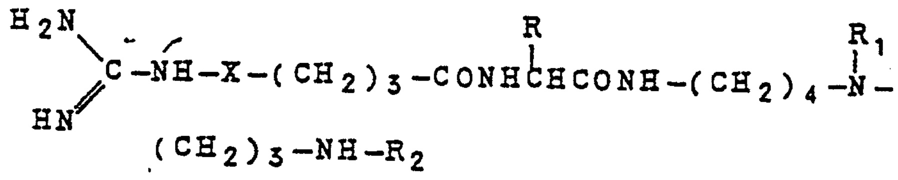

wherein X represents $-(CH_2)_{3\sim5}-$ or

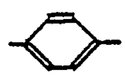 : ,

R represents -H or $-CH_2-OH$ and $R_1$ and $R_2$ each represent a residue formed by removing a hydroxyl group from the carboxyl group of an amino acid or peptide with the proviso that both of $R_1$ and $R_2$ cannot represent

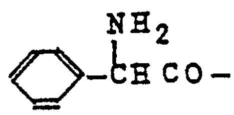

at the same time, and pharmacologically acceptable salts thereof.

2. New spergualin-related compounds and phermacologically acceptable salts thereof according to Claim 1, wherein X is

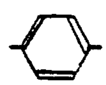,

R is $-CH_2OH$, and $R_1$ and R2 are each

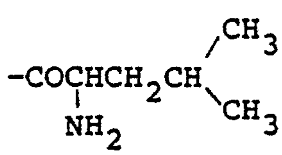.

3. An pharmaceutical composition comprising as the active ingredient a new spergualin-related compound of the following general formula [I]:

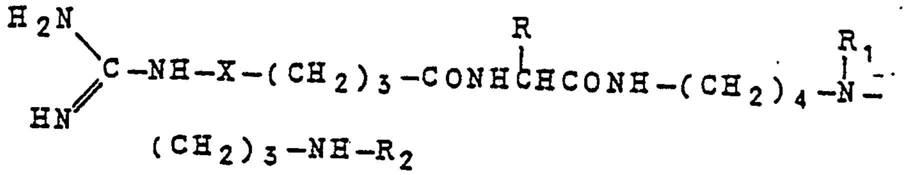

wherein X represents -$CH_2)_3$~5- or

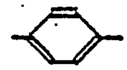 ,

R represents -H or -$CH_2$-OH, $R_1$ and $R_2$ each represent a residue formed by removing a hydroxyl group from the carboxyl group of an amino acid or peptide with the proviso that both of $R_1$ and $R_2$ cannot represent

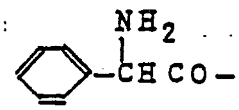

at the same time,
or a pharmacologically acceptable salt thereof.

4. An pharmaceutical composition according to Claim 3, which comprises as the active ingredient a new spergualin-related compound of the general formula [I], wherein X is

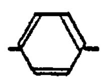 ,

R is -$CH_2OH$ and $R_1$ and $R_2$ are each

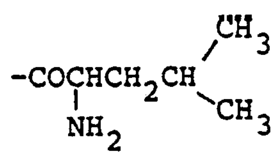

or a pharmacologically acceptable salt thereof.

5. The pharmaceutical composition according to Claim 3, which is an immunopotentiator.

FILE : 88115845.5 TABLE : 1 TAG : <<EMI NB=25 FR=1 HE=65 WI=125 TI=TAB>> OP : RLS
ORIENTATION : Portrait STUBS : Yes SEG : 1 OF : 1

Table 4

Antibody production-enhancing effect of the compound of the present invention

| Chemical | Antibody production-enhancement rate (%) (dose: 6.25 mg/kg) |
|---|---|
| Compound No. 1 | 95 |
| Physiological saline | 0 |

FILE : 88115845.5 TABLE : 3 TAG : <<EMI NB=38 FR=1 HE=210 WI=145 TI=TAB>> OP : RLS
ORIENTATION : Portrait STUBS : No SEG : 1 OF : 1

| Example | Compound of general formula [II] | Compound of general formula [I] |
|---|---|---|
| 3 | 10-{N-[4-(4-Guanidinophenyl)butyryl]-O-benzyl-L-seryl}-1,5-(di-N-benzyloxycarbonyl-L-tyrosyl-1,5,10-triazadecane hydrochloride | 10-{N-[4-(4-Guanidinophenyl)butyryl]-L-seryl}-1,5-di-L-tyrosyl-1,5,10-triazadecane trihydrochloride (Compound No. 3) |
| | NMR ($CD_3OD$) δ = 0.9 ~ 3.5 (m, 26H), 3.5 ~ 3.9 (bd, 2H, J = 5 Hz), 3.9 ~ 8.5 (b, 9H), 4.0 ~ 5.1 (m, 3H), 4.48 (s, 2H), 5.0 (s, 4H), 6.4 ~ 7.3 (q, 8H, J = 8.5 Hz), 6.9 ~ 7.5 (m, 4H), 7.25 (s, 15H). | NMR ($D_2O$, external TMS) δ = 1.4 ~ 4.0 (m, 24H), 4.1 ~ 4.4 (d, 2H, J = 5 Hz), 4.4 ~ 5.1 (m, 3H), 7.1 ~ 7.8 (q, 8H, J = 8.5 Hz), 7.4 ~ 7.9 (m, 4H). |
| | IR (KBr) ν($cm^{-1}$) = 3290, 2940, 1640, 1515, 1445, 1235. | IR (KBr) ν($cm^{-1}$) = 3240, 2940, 1635, 1510, 1365, 1230. |
| | TLC (Chloroform/methanol/17% aqueous ammonia = 6:2.5:0.5 v/v) | TLC (Chloroform/methanol/17% aqueous ammonia = 6:4:1 v/v) |
| | Rf = 0.38 | Rf = 0.12 |
| | | [α]20@D + 22.7° (C = 1.0, $H_2O$) |

COLUMN : 3

[®1α®0]<<CHF>>20@D<</CHF>> + 22.7° (C = 1.0, H®12®00)

FILE : 88115845.5 TABLE : 4 TAG : <<EMI NB=39 FR=1 HE=200 WI=145 TI=TAB>> OP : RLS
ORIENTATION : Portrait STUBS : No SEG : 1 OF : 1

| Example | Compound of general formula [II] | Compound of general formula [I] |
|---|---|---|
| 4 | 10-{N-[4-(4-Guanidinophenyl)butyryl]-O-benzyl-L-seryl}-1,5-(di-N-benzyloxycarbonyl-β-O-benzyl-L-aspartyl)-1,5,10-triazadecane hydrochloride | 10-{N-[4-(4-Guanidinophenyl)butyryl]-L-seryl}-1,5-di-L-aspartyl-1,5,10-triazadecane trihydrochloride (Compound No. 4) |
| | NMR ($CD_3OD$) δ = 1.1 ~ 3.7 (m, 24H), 3.5 ~ 3.8 (d, 2H, J = 5 Hz), 4.47 (s, 2H), 4.0 ~ 8.9 (b, 12H), 5.03 (s, 8H), 6.9 ~ 7.5 (m, 4H), 7.27 (s, 25H). | NMR ($D_2O$, external TMS) δ = 1.6 ~ 4.3 (m, 24H), 4.1 ~ 4.5 (d, 2H, J = 5 Hz), 4.6 ~ 5.0 (t, H, J = 5 Hz), 4.9 ~ 5.4 (m, 2H), 7.5 ~ 8.1 (m, 4H). |
| | IR (KBr) ν($cm^{-1}$) = 3310, 2940, 1710, 1640, 1530, 1515, 1450, 1260, 1045. | IR (KBr) ν($cm^{-1}$) = 3380, 2940, 1710, 1640, 1510, 1440, 1410, 1250. |
| | TLC (Chloroform/methanol/17% aqueous ammonia = 6:1.5:0.25 v/v) | TLC (Chloroform/methanol/17% aqueous ammonia = 6:4:1 v/v) |
| | Rf = 0.30 | Rf = 0.11 |
| | | [α]20@D − 11.2° (C = 1.0, $H_2O$) |

COLUMN : 3

[@1α@0]<<CHF>>20@D<</CHF>> @1−@0 11.2° (C = 1.0, H@12@00)

FILE : 88115845.5 TABLE : 5 TAG : <<EMI NB=40 FR=1 HE=210 WI=145 TI=TAB>> OP : RLS
ORIENTATION : Portrait STUBS : No SEG : 1 OF : 1

| Example | Compound of general formula [II] | Compound of general formula [I] |
|---|---|---|
| 5 | 10-{N-[4-(4-Guanidinophenyl)butyryl]-O-benzyl-L-seryl}-1,5-(di-N-benzyloxycarbonyl-L-alanyl)-1,5,10-triazadecane hydrochloride | 10-{N-[4-(4-Guanidinophenyl)butyryl]-L-seryl}-1,5-di-L-alanyl-1,5,10-triazadecane trihydrochloride (Compound No. 5) |
| | NMR ($CD_3OD$) δ = 1.0 ~ 3.4 (m, 26H), 3.4 ~ 3.7 (d, J = 5 Hz, 2H), 3.7 ~ 4.1 (m, 3H), 4.3 (s, 2H), 4.9 (s, 4H), 3.5 ~7.5 (m, 9H), 6.8 ~ 7.3 (m, 4H), 7.1 (s, 15H). | NMR ($D_2O$, external TMS) δ = 1.8 ~ 2.1, (d, J = 7 Hz, 3H), 1.9 ~ 2.2 (d, J = 7 Hz, 3H), 2.2 ~ 4.1 (m, 20H), 4.1 ~ 4.4 (d, J = 5 Hz, 2H), 4.4 ~ 5.0 (m, 3H), 7.5 ~ 8.0 (m, 4H). |
| | IR (KBr) ν($cm^{-1}$) = 3280, 2930, 1700, 1635, 1510, 1445, 1243, 1095, 1060, 1020. | IR (KBr) ν($cm^{-1}$) = 3300, 3000, 1670, 1645, 1420, 1360, 1240, 1200, 1100. |
| | TLC (Chloroform/methanol/17% aqueous ammonia = 6:1.5:0.25 v/v) | TLC (Chloroform/methanol/17% aqueous ammonia = 2:1:1 v/v) |
| | Rf = 0.47 | Rf = 0.40 |
| | | [α]20@D − 10.6° (C = 0.97, $H_2O$) |

COLUMN : 3

[@1α@0]<<CHF>>20@D<</CHF>> @1−@0 10.6° (C = 0.97, H@12@00)

FILE : 88115845.5 TABLE : 6 TAG : <<EMI NB=41 FR=1 HE=210 WI=145 TI=TAB>> OP : RLS
ORIENTATION : Portrait STUBS : No SEG : 1 OF : 1

| Example | Compound of general formula [II] | Compound of general formula [I] |
|---|---|---|
| 6 | 10-{N-[4-(4-Guanidinophenyl)butyryl]-O-benzyl-L-seryl}-1,5-(di-N-benzyloxycarbonyl-O-benzyl-L-seryl)-1,5,10-triazadecane hydrochloride | 10-{N-[4-(4-Guanidinophenyl)butyryl]-L-seryl}-1,5-di-L-seryl-1,5,10-triazadecane trihydrochloride (Compound No. 6) |
| | NMR ($CD_3OD$) δ = 1.2 ~ 3.5 (m, 20H), 3.5 ~ 3.8 (d, J = 5 Hz, 6H), 4.1 ~ 4.6 (m, 3H), 4.4 (s, 6H), 4.9 ~ 5.1 (s, 4H), 4.1 ~ 7.5 (m, 9H), 6.9 ~ 7.5 (m, 4H), 7.2 (s, 25H). | NMR ($D_2O$, external TMS) δ = 1.5 ~ 4.1 (m, 20H), 4.1 ~ 4.6 (m, 6H), 4.6 ~ 5.2 (m, 3H), 7.5 ~ 8.0 (m, 4H). |
| | IR (KBr) ν($cm^{-1}$) = 3300, 3050, 2925, 2850, 1710, 1615, 1510, 1425, 1360, 1240, 1100, 1020. | IR (KBr) ν($cm^{-1}$) = 3300, 2925, 1635, 1500, 1360, 1250, 1050. |
| | TLC (Chloroform/methanol/17% aqueous ammonia = 6:1.5:0.25 v/v) | TLC (Propanol/pyridine/water/acetic acid = 6:4:3:2 v/v) |
| | Rf = 0.41 | Rf = 0.36 |
| | | [α]20@D − 9.6° (C = 0.98, $D_2O$) |

COLUMN : 3

[@1a@0]<<CHF>>20@D<</CHF>> @1–@0 9.6° (C = 0.98, D@12@00)

FILE : 88115845.5 TABLE : 7 TAG : <<EMI NB=42 FR=1 HE=195 WI=145 TI=TAB>> OP : RLS
ORIENTATION : Portrait STUBS : No SEG : 1 OF : 1

| Example | Compound of general formula [II] | Compound of general formula [I] |
|---|---|---|
| 7 | 10-{N-[4-(4-Guanidinophenyl)butyryl]-O-t-butyl-L-seryl}-1,5-(di-N-t-butoxycarbonyl-L-prolyl)-1,5,10-triazadecane hydrochloride | 10-{N-[4-(4-Guanidinophenyl)butyryl]-L-seryl}-1,5-di-L-prolyl-1,5,10-triazadecane trihydrochloride (Compound No. 7) |
| | NMR ($CD_3OD$) δ = 0.9 ~ 4.0 (m, 32H), 1.3 (s, 9H), 1.5 (s, 18H), 4.0 ~ 4.7 (m, 3H), 4.0 ~ 7.6 (m, 9H), 7.0 ~ 7.6 (m, 4H). | NMR ($D_2O$, external TMS) δ = 1.8 ~ 4.2 (m, 32H), 4.2 ~ 4.5 (d, J = 5 Hz, 2H), 4.6 ~ 5.2 (m, 3H), 7.6 ~ 8.0 (m, 4H). |
| | IR (KBr) ν($cm^{-1}$) = 3300, 3010, 1690, 1570, 1540, 1500, 1430, 1400, 1280, 1190, 1145, 1100. | IR (KBr) ν($cm^{-1}$) = 3300, 3075, 2930, 1660, 1640, 1550, 1510, 1440, 1370, 1250. |
| | TLC (Chloroform/methanol/17% aqueous ammonia = 6:1.5:0.25 v/v) | TLC (Propanol/pyridine/water/acetic acid = 6:4:3:2 v/v) |
| | Rf = 0.34 | Rf = 0.43 |
| | | [α]20@D − 51.8° (C = 1.0, $H_2O$) |

COLUMN : 3

[@1α@0]<<CHF>>20@D<</CHF>> @1−@0 51.8° (C = 1.0, H@12@00)

FILE : 88115845.5 TABLE : 8 TAG : <<EMI NB=43 FR=1 HE=210 WI=145 TI=TAB>> OP : RLS
ORIENTATION : Portrait STUBS : No SEG : 1 OF : 1

| Example | Compound of general formula [II] | Compound of general formula [I] |
|---|---|---|
| 8 | 10-{N-[4-(4-Guanidinophenyl)butyryl]-O-benzyl-L-seryl}-1,5-(di-N-benzyloxycarbonyl-β-alanyl)-1,5,10-triazadecane hydrochloride | 10-{N-[4-(4-Guanidinophenyl)butyryl]-L-seryl}-1,5-di-β-analyl-1,5,10-triazadecane trihydrochloride (Compound No. 8) |
| | NMR ($CD_3OD$) δ = 0.9 ~ 3.8 (m, 38H), 1.1 ~ 1.4 (d, J = 7Hz, 6H), 4.5 (s, 2H), 4.4 ~ 4.7 (m, 1H), 5.0 (s, 4H), 3.6 ~ 7.6 (m, 9H), 6.9 ~ 7.4 (m, 4H), 7.2 (s, 15H). | NMR ($D_2O$, external TMS) δ = 1.7 ~ 4.1 (m, 32H), 4.1 ~ 4.4 (d, J = 5 Hz, 2H), 4.6 ~ 5.0 (m, 1H), 7.5 ~ 8.1 (m, 4H). |
| | IR (KBr) ν($cm^{-1}$) = 3275, 2925, 2850, 1700, 1610, 1500, 1440, 1360, 1240, 1100, 1060. | IR (KBr) ν($cm^{-1}$) = 3375, 2940, 1615, 1545, 1510, 1455, 1380, 1315, 1250, 1200, 1090. |
| | TLC (Chloroform/methanol/17% aqueous ammonia = 6:1.5:0.25 v/v) | TLC (Propanol/pyridine/water/acetic acid = 6:4:3:2 v/v) |
| | Rf = 0.47 | Rf = 0.24 |
| | | [α]20@D − 10.1° (C = 1.0, $H_2O$) |

COLUMN : 3

[@1α@0]<<CHF>>20@D<</CHF>> @1−@0 10.1° (C = 1.0, H@12@00)

FILE : 88115845.5 TABLE : 9 TAG : <<EMI NB=44 FR=1 HE=205 WI=145 TI=TAB>> OP : RLS
ORIENTATION : Portrait STUBS : No SEG : 1 OF : 1

| Example | Compound of general formula [II] | Compound of general formula [I] |
|---|---|---|
| 9 | 10-{N-[4-(4-Guanidinophenyl)butyryl]-O-benzyl-L-seryl}-1,5-(di-N-benzyloxycarbonyl-L-glycyl)-1,5,10-triazadecane hydrochloride | 10-{N-[4-(4-Guanidinophenyl)butyryl]-L-seryl}-1,5-di-L-glycyl-1,5,10-triazadecane trihydrochloride (Compound No. 9) |
| | NMR ($CD_3OD$) δ = 0.8 ~ 3.9 (m, 24H), 3.9 ~ 4.1 (m, 2H), 4.3 ~ 4.6 (m, 3H), 5.0 (s, 4H), 4.2 ~ 7.5 (m, 9H), 7.0 ~ 7.5 (m, 4H), 7.2 (s, 15H). | NMR ($D_2O$, external TMS) δ = 1.7 ~ 4.1 (m, 24H), 4.1 ~ 4.4 (d, J = 6 Hz, 4H), 4.4 ~ 4.7 (d, J = 5 Hz, 2H), 4.7 ~ 5.0 (m, 1H), 7.6 ~ 8.0 (m, 4H). |
| | IR (KBr) $\nu(cm^{-1})$ = 3300, 3070, 2900, 1605, 1520, 1430, 1225, 1105, 1050. | IR (KBr) $\nu(cm^{-1})$ = 3250, 3075, 2930, 1642, 1555, 1518, 1503, 1415, 1360, 1250, 1212, 1070. |
| | TLC (Chloroform/methanol/17% aqueous ammonia = 6:1.5:0.25 v/v) | TLC (Chloroform/methanol/17% aqueous ammonia = 2:1:1 v/v) |
| | Rf = 0.42 | Rf = 0.26 |
| | | [α]20@D − 19.4° (C = 1.0, $H_2O$) |

COLUMN : 3

[@1α@0]<<CHF>>20@D<</CHF>> @1−@0 19.4° (C = 1.0, H@12@00)

FILE : 88115845.5 TABLE : 10 TAG : <<EMI NB=45 FR=1 HE=205 WI=145 TI=TAB>> OP : RLS
ORIENTATION : Portrait STUBS : No SEG : 1 OF : 1

| Example | Compound of general formula [II] | Compound of general formula [I] |
|---|---|---|
| 10 | 10-{N-[4-(4-Guanidinophenyl)butyryl]-O-benzyl-L-seryl)-1,5-(di-N-benzyloxycarbonyl-L-glutaminyl)-1,5,10-triazadecane hydrochloride | 10-{N-[4-(4-Guanidinophenyl)butyryl]-L-seryl)-1,5-di-L-glutaminyl-1,5,10-triazadecane trihydrochloride (Compound No. 10) |
| | NMR ($CD_3OD$) δ = 1.3 ~ 5.2 (m, 30H), 3.7 ~ 3.9 (m, 2H), 3.9 ~ 4.3 (m, 1H), 4.5 (s, 2H), 5.1 (s, 4H), 3.5 ~ 7.5 (m, 13H), 6.9 ~ 7.5 (m, 4H), 7.3 (s, 15H). | NMR ($D_2O$, external TMS) δ = 1.6 ~ 4.1 (m, 28H), 4.1 ~ 4.4 (d, J = 5 Hz, 2H), 4.5 ~ 4.9 (m, 3H), 7.5 ~ 7.9 (m, 4H). |
| | IR (KBr) ν($cm^{-1}$) = 3300, 2920, 2880, 1640, 1550, 1440, 1250, 1200, 1125, 1045. | IR (KBr) ν($cm^{-1}$) = 3230, 2930, 2460, 1640, 1540, 1520, 1440, 1370, 1250, 1200, 1150, 1060, 1030. |
| | TCLC (Chloroform/methanol/17% aqueous ammonia = 6:1.5:0.25 v/v) | TLC (Chloroform/methanol/17% aqueous ammonia = 2:1:1 v/v) |
| | Rf = 0.19 | Rf = 0.30 |
| | | [α]20@D − 17.5° (C = 1.04, $H_2O$) |

COLUMN : 3

[@1α@0]<<CHF>>20@D<</CHF>> @1−@0 17.5° (C = 1.04, H@12@00)

FILE : 88115845.5 TABLE : 11 TAG : <<EMI NB=46 FR=1 HE=195 WI=145 TI=TAB>> OP : RLS
ORIENTATION : Portrait STUBS : No SEG : 1 OF : 1

| Example | Compound of general formula [II] | Compound of general formula [I] |
|---|---|---|
| 11 | 10-{N-[4-(4-Guanidinophenyl)butyryl]-O-benzyl-L-seryl)-1,5-(di-N-benzyloxycarbonyl-L-lysyl)-1,5,10-triazadecane hydrochloride | 10-{N-[4-(4-Guanidinophenyl)butyryl]-L-seryl}-1,5-di-L-lysyl-1,5,10-triazadecane trihydrochloride (Compound No. 11) |
| | NMR ($CD_3OD$) $\delta$ = 1.0 ~ 3.5 (m, 26H), 3.5 ~ 3.8, (d, J = 5 Hz, 2H), 4.5 (s, 2H), 5.0 (s, 4H), 3.5 ~ 5.1 (m, 6H), 3.5 ~ 7.4 (m, 13H), 6.9 ~ 7.4, (m, 4H), 7.3 (s, 15H). | NMR ($D_2O$, external TMS) $\delta$ = 1.7 ~ 3.8 (m, 32H), 4.1 ~ 4.3 (d, J = 5 Hz, 2H), 4.1 ~ 5.1 (m, 7H), 7.5 ~ 7.9 (m, 4H). |
| | IR (KBr) $\nu(cm^{-1})$ = 3300, 3060, 2930, 2850, 1680, 1630, 1520, 1445, 1224, 1100, 1020, 1010. | IR (KBr) $\nu(cm^{-1})$ = 3380, 3050, 2940, 1990, 1615, 1505, 1370, 1250, 1115, 1060. |
| | TLC (Chloroform/methanol = 10:1 v/v) | TLC (n-Propanol/pyridine/water/acetic acid = 6:4:3:2 v/v) |
| | Rf = 0.18 | Rf = 0.14 |
| | | [α]20@D + 21.7° (C = 0.97, $H_2O$) |

COLUMN : 3

[@1α@0]<<CHF>>20@D<</CHF>> + 21.7° (C = 0.97, H@12@00)